(19) Europäisches Patentamt
European Patent Office
Office européen des brevets

(11) EP 2 181 710 A1

(12) **EUROPEAN PATENT APPLICATION**

(43) Date of publication:
**05.05.2010 Bulletin 2010/18**

(21) Application number: **08018795.8**

(22) Date of filing: **28.10.2008**

(51) Int Cl.:
***A61K 31/575*** (2006.01) ***A61P 17/06*** (2006.01)
***A61P 37/06*** (2006.01) ***A61P 1/00*** (2006.01)
***A61P 17/00*** (2006.01) ***A61P 29/00*** (2006.01)
***G01N 33/50*** (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Phenex Pharmaceuticals AG**
**67056 Ludwigshafen (DE)**

(72) Inventors:
- **Kremoser, Claus**
  **69121 Heidelberg (DE)**
- **Abel, Ulrich**
  **69126 Heidelberg (DE)**
- **Deutschle, Ulrich**
  **67549 Worms (DE)**
- **Schlueter, Thomas**
  **69123 Heidelberg (DE)**

(74) Representative: **Grünecker, Kinkeldey, Stockmair & Schwanhäusser**
**Anwaltssozietät**
**Leopoldstrasse 4**
**80802 München (DE)**

(54) **Ligands for modulation of orphan receptor-gamma (NR1F3) activity**

(57) The invention relates to modulators for the orphan nuclear receptor RORgamma and methods for identification and screening of novel modulators for RORgamma activity as well as methods for treating RORgamma mediated diseases with novel RORgamma modulators identified by such methods.

EP 2 181 710 A1

## Description

The invention provides modulators for the orphan nuclear receptor RORgamma and methods for identification and screening of novel modulators for RORgamma activity and methods for treating RORgamma mediated diseases with novel RORgamma modulators identified by such methods.

The retinoid-receptor related orphan receptors consist of three family members, namely RORα (Becker-Andree, Biochem. Biophys. Res. Commun. 1993, 194:1371-1379), RORβ (Andre et al., Gene 1998, 516:277-283) and RORγ (He et al, Immunity 1998, 9:797-806) and constitute the NR1F (ROR/RZR) subgroup of the nuclear receptor superfamily (Mangelsdorf et al., Cell 1995, 83:835-839).

The nuclear receptor superfamily shares common modular structural domains consisting of a hypervariable N-terminal domain, a conserved DNA binding domain (DBD), a hinge region, and a conserved ligand-binding domain (LBD). The DBD targets the receptor to specific DNA sequences (nuclear hormone response elements or NRE's), and the LBD functions in the recognition of endogenous or exogenous chemical ligands. A constitutive transcriptional activation domain is found at the N-terminus (AF1) and a ligand regulated transcriptional activation domain is embedded within the C-terminal LBD of typical NR's. The nuclear receptors can exist in a transcriptional activating or repressing state when bound to their target NRE's. The basic mechanism of gene activation involves ligand dependent exchange of co-regulatory proteins, namely co-activators and co-repressors (McKenna et al., Endocrine Rev. 1999, 20:321-344). A NR in the repressing state is bound to its DNA recognition element and is associated with co-repressor proteins that recruit histonme-deacetylases (HDACS). In the presence of an agonist, co-repressors are exchanged for coactivators that recruit transcription factors, which contribute to assembling of a chromatin-remodelling complex, which via histone acetylation relieves transcriptional repression and stimulates transcriptional initiation. The AF-2 domain of the LBD acts as a ligand dependant molecular switch presenting interaction surfaces for co-repressor or co-activator proteins and providing with a conserved mechanism for gene activation or repression that is shared by the members of the nuclear receptor superfamily.

The members of the NR1F family of nuclear receptors (such as RORgamma) are considered as constitutive active transcription factors in absence of known ligand, which is similar to the estrogen-related receptor alpha (Vanacker et al., Mol. Endocrinol. 1999, 13:864-773). For ERRalpha, synthetic inverse agonists have been described that reduce ERRalpha transcriptional activity by interfering with ERRalpha/PGC1 alpha signalling (Willy et al., PNAS 2004, 101: 8912-8917). It can be expected that inverse agonists of RORgamma, for instance, should reduce the transcriptional activity of RORgamma and in a functional negative way influence the biological pathways controlled by RORgamma.

The RORs are expressed as isoforms arising from differential splicing or alternative transcriptional start sites. So far, isoforms have been described that differ only in their N-terminal domain (A/B-domain). In humans, four different RORα isoforms have been identified (RORα1-4) while only two isoforms are known for both RORβ (1 and 2) and RORγ (1 and 2) (Andre et al., Gene 1998, 216:277-283; Villey et al., Eur. J. Immunol. 1999, 29:4072-7080). RORgamma is used as a term describing both, RORγ1 and/or RORγ2.

The ROR isoforms show different tissue expression patterns and regulate different target genes and physiological pathways. For example, the RORγ2 (also called RORγ-t) is highly restricted to $CD4^+CD8^+$ thymocytes while other tissues express RORγ1 (Eberl et al., Science 2004, 305:248-251).

RORs exhibit a structural architecture that is typical of nuclear receptors. RORs contain four major functional domains: an amino-terminal (A/B) domain, a DNA-binding domain (DBD), a hinge domain, and a ligand-binding domain (LBD) (Evans et al., Science 1988, 240:889-895). The DBD consists of two highly conserved zinc finger motifs involved in the recognition of ROR response elements (ROREs) which consist of the consensus motif AGGTCA preceded by an AT-rich sequence (Andre et al., Gene 1998, 216:277-283) which is similar to that of the nuclear receptors Rev-ErbAa and Rev-Erbβ (NR1D1 and D2, respectively) (Giguere et al., Genomics 1995, 28:596-598). These recognition elements do also show high similarity to those identified for the estrogen related receptors and in particular ERRα (ERRs, NR3B1, -2, -3) (Vanacker et al., Mol. Endocrinol. 1999, 13:764-773), steroidogenic factor 1 (SF-1, NR5A) and NGFI-B (NR4A1, -2, -3) (Wilson et al., Mol. Cell Biol. 1993, 13:5794-5708).

The Rev-Erb receptors act as constitutive transcriptional repressors, and since they bind to similar DNA recognition sequences, they are able to inhibit ROR-mediated transcriptional activation by competing with RORs for the very same DNA response element (Forman et al. Cross-talk among ROR alpha 1 and the Rev-erb family of orphan nuclear receptors. Mol. Endocrinol. 8:1253-1261, 1994). A physiological significance of such an interplay is evident in the control of circadian rhythm, where Rev-Erb and RORα do repress and activate transcription of the Bmal1 transcription factor, respectively, that plays an important role in the control of the circadian clock (Akashi and Takumi, Nat. Struct. Mol. Biol. 2005, 12:441-448). Such cross-talks between a family member of the RORs and other nuclear receptors binding to similar recognition elements like the ERRα may operate in the control of other physiological pathways as well.

RORα is highly expressed in different brain regions and most highly in cerebellum and thalamus. RORα knock-out mice show ataxia with strong cerebellar atrophy which is highly similar to the symptoms displayed in the so-called staggerer mutant mouse ($ROR\alpha^{sg/sg}$) which carries mutations in RORα that results in a truncated RORα which does

not contain a LBD (Hamilton et al., Nature 1996, 379:736-739).

Analysis of RORα$^{sg/sg}$ staggerer-mice have in addition revealed a strong impact on lipid metabolism, namely significant decreases in serum and liver triglyceride, reduced serum HDL cholesterol levels and reduced adiposity. SREBP1c and the cholesterol transporters ABCA1 and ABCG1 are reduced in livers of staggerer mice and CHIP analysis suggest that RORα is directly recruited to and regulates the SREBP1c promoter. In addition, PGC1α, PGC1β, lipin1 and β2-adrenergic receptor were found to be increased in tissues such as liver or white and brown adipose tissue, which may help to explain the observed resistance to diet-induced obesity in staggerer mice (Lau et al., J. Biol. Chem. 2008, 283:18411-18421).

RORβ is more differentially expressed, namely in certain regions of the brain and in the retina. RORβ knock-out mice display a duck-like gait and retinal degeneration which leads to blindness (Andre et al., EMBO. J. 1998, 17: 3867-3877). The molecular mechanisms behind this retinal degeneration are still poorly understood.

RORγ (particularly RORγ2) null-mutant mice lack lymph nodes and Peyer's patches (Eberl and Littmann, Immunol. Rev. 2003, 195:81-90) and lymphatic tissue inducer (LTi) cells are completely absent from spleen mesentery and intestine. In addition, the size of the thymus and the number of thymocytes is greatly reduced in RORγ null mice (Sun et al., Science 2000, 288:2369-2373) due to a reduction in double-positive $CD4^+CD8^+$ and single positive $CD4^-CD8^+$ or $CD4^+CD8^-$ cells suggesting a very important role of RORγ2 in thymocyte development.

Thymocyte development follows a complex program involving coordinated cycles of proliferation, differentiation, cell death and gene recombination in cell populations dedicated by their microenvironment. Pluripotent lymphocyte progenitors migrating from fetal liver or adult bone marrow to the thymus are being committed to the T-cell lineage. They develop through a series of steps from CD4-CD8- double negative cells to $CD4^+CD8^+$ cells and those with low affinity towards self-MHC peptides are eliminated by negative selection. These develop into $CD4^-CD8^+$ (killer) or $CD4^+CD8^-$ (helper) T-cell lineages. RORγ2 is not expressed in double negative and little expressed in immature single negative thymocytes (He et al., J. Immunol. 2000, 164:5668-5674), while highly upregulated in double-positive thymocytes and downregulated during differentiation in single-positive thymocytes. RORγ deficiency results in increased apoptosis in $CD4^+CD8^+$ cells and the number of peripheral blood thymocytes is decreased by 6-fold (10-fold $CD4^+$ and 3-fold $CD8^+$ thymocytes).

Recent experiments in a model of ovalbumin (OVA)-induced inflammation in mice, as a model for allergic airway disease, demonstrated a severe impairment of the development of the allergic phenotype in the RORγ KO mice with decreased numbers of $CD4^+$ cells and lower Th2 cytokine/chemokine protein and mRNA expression in the lungs after challenge with OVA (Tilley et al., J. Immunol. 2007, 178:3208-3218). IFN-γ and IL-10 production were increased in splenocytes following re-stimulation with the OVA antigen compared to wt splenocytes suggesting a shift towards a Th1 type immune response on cost of a reduction of Th2 type response. This suggests that down-modulation of RORγ transcriptional activity with a ligand could result in a similar shift of the immune response towards a Th2 type response, which could be beneficial in the treatment of certain allergic inflammatory conditions.

T-helper cells were previously considered to consist of Th1 and Th2 cells. However, a new class of Th cells, the Th17 cells, which produce IL-17, were identified as a unique class of T-cells that are considered to be pro-inflammatory. They are recognized as key players in autoimmune and inflammatory diseases since IL-17 expression has been associated with many inflammatory diseases such as rheumatoid arthritis, systemic lupus erythematosis (SLE) and allograft rejection. (Tesmer et al. Immunol. Rev. 2008, 223:87-113).

RORγ2 is exclusively expressed in cells of the immune system and has been identified as a master regulator of Th17 cell differentiation. Expression of RORγ2 is induced by TGF-beta or IL-6 and overexpression of RORγ2 results in increased Th17 cell lineage and IL-17 expression. RORγ2 KO mice show very little Th17 cells in the intestinal lamina propria but Th17 cells are still detectable. Recently Yang et al. (2008) reported the expression of RORα in Th17 cells, which is regulated by TGF-beta and IL-6 in a STAT3 dependent fashion. Double mutations in RORα and RORγ2 completely inhibited Th17 differentiation in vitro and in vivo and completely blocked the development of symptoms in a model of EAE (experimental autoimmune encephalitis) (Yang et al., Immunity 2008, 28:29-39). This suggests that RORγ2 and RORα synergistically control Th17 development. Inhibitors of both RORγ2 and RORα may inhibit the development of Th17 cells and the expression of pro-inflammatory IL-17 in inflammatory diseases. Inhibition of IL-17 production via inhibition of Th17 cell development may also be advantageous in atopic dermatitis and psoriasis where IL-17 is deeply involved. Interestingly, recent evidence was presented that IL-10 suppresses the expression of IL-17 secreted by both, macrophages and T-cells. In addition, the expression of the Th17 transcription factor RORγ2 was suppressed (Gu et al., Eur. J. Immunol. 2008, 38:1807-1813). Moreover, IL-10 deficient mice provide a good model for Inflammatory Bowel Disease (IBD) where a shift towards a Th1 type inflammatory response is frequently observed. Oral IL-10 delivery poses a potential treatment option for IBD.

RORγ1 is expressed in muscle and several other tissues including pancreas, thymus, prostate, liver and testis. Ectopic overexpression of dominant active and dominant negative versions of RORγ1 showed that this receptor controls genes involved in lipid metabolism (FABP4, CD36, LPL and UCP3), cholesterol efflux (ABCA1, ABCG1) (carbohydrate metabolism (GLUT5, adiponectin receptor 2 and IL-15) and muscle mass (myostatin and IL-15).

RORα1 and RORγ1 are expressed in liver and oscillate in a circadian fashion. Double KO mice show that these genes are involved in regulation of phase I and phase II metabolic enzymes including 3-beta-hydroxysteroid dehydrogenases, Cyp450 enzymes and sulfotransferases, suggesting important roles in steroid, bile acid and xenobiotic metabolisms (Kang et al. Physiol. Genomics 2007, 31:281-294). One of the genes regulated was shown to be Cyp7b1, which has an important role in cholesterol metabolism and it was shown that RORα is necessary and sufficient for regulation of Cyp7b1. Analysis of target genes in RORα and LXRα KO mice raised the hypothesis that both receptors are mutually suppressive with respective to their target genes (Wada et al., Exp. Biol. Medicine 2008, epub).

<u>Ligands for the RORs:</u>

It was reported that cholesterol and its sulfated derivatives might function as RORα ligands and in particular cholesterol-sulfate could restore transcriptional activity of RORα in cholesterol-depleted cells (Kallen et al., Structure 2002, 10:1697-1707). Previously, melatonin (Missbach et al., J. Biol. Chem. 1998, 271:13515-13522) and thiazolidindiones were suggested to bind to RORα (Wiesenberg et al., Nucleic Acid Res. 1995, 23:327-333). However, none of these have been shown to be functional ligands of RORα or any of the other RORs. Certain retinoids including all-trans retinoid acid have been demonstrated to bind to RORβ and function as partial antagonists for RORβ but not RORα (Stehlin-Gaon et al., Nat. Struct. Biol. 2003, 10:820-825). However, none of these or any other ligands have been described yet to bind to and/or modulate the transcriptional activity of RORγ1 or RORγ2 despite the cloning of the mouse and human RORgamma cDNAs as a potential basis for generating methods for screening of substance libraries for RORgamma modulating compounds (Medvedev et al., Gene 1996, 181:199-206, WO 2000/24757).

It is therefore the object of the present invention to provide compounds, which bind to the orphan nuclear receptors RORγ1 and/or RORγ2 and, thus, to open new methods for treating diseases associated with the modulation of RORgamma, such as autoimmune diseases, inflammatory skin diseases or multiple sclerosis.

It is further the object of the present invention to provide a screening method for identifying the ligands of RORgamma and for measuring the activity of said ligands.

This object is solved by the surprising discovery of small molecule ligands for the human RORgamma. Among these ligands are retinoids, but also rexinoids.

Thus, the present invention provides RORgamma modulators, which can be used for treating or preventing a disease or disorder associated with the inactivation or activation of the RORgamma receptor.

The present invention further provides a method for modulating RORgamma activity in a cell culture or in a biochemical cell-free *in vitro* assay system comprising the step of administrating to such cell culture or assay system an effective amount of a RORgamma modulator as described herein, sufficient to induce or reduce the readout of RORgamma activity in such cell culture or biochemical assay system.

Furthermore, the present invention relates to compounds identified by the methods described herein.

**Brief description of the figures**

Figure 1 shows the results of a radioligand displacement assay, wherein the replacement of radioactive $H^3$-25-hydroxycholesterol, bound to the RORgamma-ligand binding domain (RORγ-LBD), by the compounds described herein is measured.

Figure 2 shows the structural formulas of the compounds used in the present invention.

The present invention relates to a RORgamma modulator for use in the treatment or prophylaxis of a disease or disorder associated with the inhibition or activation of a RORgamma receptor.

When treating the disease or disorder associated with the modulation of the RORgamma receptor, the activity of said receptor is preferably reduced.

Preferably, the disease or disorder is selected from the group consisting of autoimmune diseases, inflammatory skin diseases and multiple sclerosis.

The RORgamma modulator used in the present invention preferably comprises a compound of formula (I) of (II).

The compounds of formula (I) have the following structure

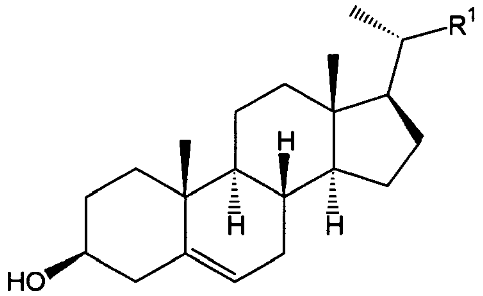

(I)

or a solvate or a pharmaceutically acceptable salt thereof, wherein

$R^1$ is

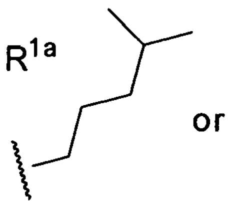

or

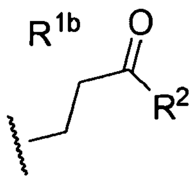

or

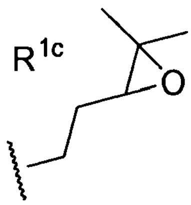

wherein each $R^{1a}$, $R^{1b}$ or $R^{1c}$ is optionally substituted with one or two hydroxy groups,
$R^2$ is $OR^3$ or $NR^3R^4$, and
$R^3$ and $R^4$ are independently from each other selected from hydrogen or $C_1$-$C_3$alkyl.

The compounds of formula (II) have the following structure

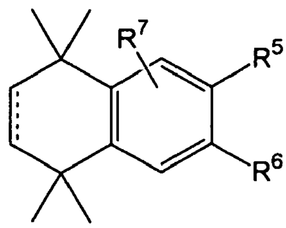

(II)

or a solvate or a pharmaceutically acceptable salt thereof, wherein

$R^5$ is $CONHR^8$, $NHCOR^8$, $C(O)R^8$, $CH=CHR^8$, $C(CH_3)=CHR^8$, $C{\equiv}CR^8$, $CH(OH)CH=CHR^8$, $C(O)CH=CHR^8$, 5 to 6-membered heterocyclyl-$R^8$,
$R^6$ is hydrogen,
$R^5$ and $R^6$ may also together form

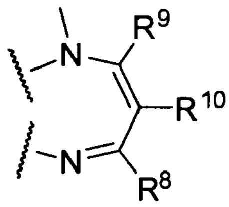

$R^7$ is hydrogen, fluorine, chlorine or hydroxy,
$R^8$ is 4-yl-benzoic acid or 6-yl-2-naphthoic acid, and
$R^9$ and $R^{10}$ are hydrogen or $R^9$ and $R^{10}$ form together with the bond to which they attach a fused 5 to 10-membered heteroaromatic or aromatic monocyclic or bicyclic ring.

In the above and the following, the employed terms have independently the meaning as described below:

A 5 to 10-membered aromatic mono- or bicyclic moiety is preferably selected from phenyl, biphenyl, naphthyl, tetrahydronaphthyl, fluorenyl, indenyl and phenanthrenyl, more preferably phenyl and naphthyl.

A 5 to 10-membered heteroaromatic monocyclic or bicyclic ring is a ringsystem having 4 to 9 carbon atoms and at least one ring containing at least one heteroatom selected from O, N and/or S. Preferably, heteroaryl contains 1, 2, 3 or 4, more preferably 1, 2 or 3 heteroatoms selected from O and/or N and is preferably selected from pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl and furopyridinyl. Spiro moieties are also included within the scope of this definition. Preferred heteroaryl includes pyridinyl, imidazolyl, pyrimidinyl, pyrazolyl, triazolyl, pyrazinyl, tetrazolyl, isoxazolyl, oxazolyl, isothiazolyl, oxadiazolyl and triazolyl.

Heterocyclyl is a 5 to 6-membered saturated or unsaturated ring containing at least one heteroatom selected from O, N and/or S and 1, 2, 3, 4, or 5 carbon atoms. Preferably, heterocyclyl contains 1, 2, 3 or 4, more preferably 1, 2 or 3 heteroatoms selected from O and/or N. Heterocyclyl includes mono- and bicyclic ringsystems and is preferably selected from pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, azetidin-2-one-1-yl, pyrrolidin-2-one-1-yl, piperid-2-one-1-yl, azepan-2-one-1-yl, 3-azabicyco[3.1.0] hexanyl, 3-azabicyclo[4.1.0]heptanyl, azabicyclo[2.2.2]hexanyl, 3H-indolyl and quinolizinyl. Spiromoieties are also included within the scope of this definition.
Preferred embodiments of the compounds used in the present invention are shown in Figure 2.
The compounds used in the present invention can be in the form of a pharmaceutically acceptable salt or a solvate. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the present invention which contain acidic groups can be present on these groups and can be used according to the invention, for example, as alkali metal salts, alkaline earth metal salts or ammonium salts. More precise examples of such salts include sodium salts, potassium salts, calcium salts, magnesium salts or salts with ammonia or organic amines such as, for example, ethylamine, ethanolamine, triethanolamine or amino acids. The compounds of the present invention which contain one or more basic groups, i.e. groups which can be protonated, can be present and can be used according to the invention in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain

acidic and basic groups in the molecule, the invention also includes, in addition to the salt forms mentioned, inner salts or betaines (zwitterions). The respective salts can be obtained by customary methods which are known to the person skilled in the art like, for example, by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

In practical use, the compounds used in the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

The compounds used in the present invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the present invention are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing RORgamma-mediated conditions for which compounds of formula (I) and (II) are indicated, generally satisfactory results are obtained when the compounds are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligram to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

For the first time, the present invention describes modulators, in the following also referred to as ligands, which bind to the RORgamma receptor. Surprisingly, it has been found that certain oxysterols such as compounds of formula (I) and certain synthetic retinoids such as compounds of formula (II) act as modulators of the RORgamma receptor.

The RORgamma receptor is considered to be involved in thymocyte development, thus the modulators described herein may be useful in the treatment of inflammatory skin diseases such as atopic eczema and psoriasis. It is further suggested that down-modulation of RORγ transcriptional activity with a ligand could result in a shift of the immune response towards a Th2 type response which could be beneficial in the treatment of certain allergic inflammatory conditions such as rheumatoid arthritis, systemic lupus erythomatosis, inflammatory bowel disease (Crohn's Disease), and multiple sclerosis (Tesmer et. al., Immunol. Rev. 2008, 223:97-113)

The compounds of formula (I) and (II) show agonistic and antagonistic activity, respectively, with respect to the dose dependent modulation of the constitutive interaction of the RORγ ligand binding domain with peptides derived from the co-activators such as SRC-1 or TIF-2.

It has been surprisingly found that the interaction between RORγ ligand binding domain and the peptides can be determined by a homogenous FRET based ligand-sensing assays. Even more surprising was the identification of oxysterols, like 22-R-hydroxycholesterol, 25-R-hydroxycholesterol, 27-hydroxycholesterol and the semisynthetic oxysterols 5-cholenic acid-methyl-ester-3β-hydroxycholenamide (CHAME) and N,N-dimethyl-3-hydroxycholenamide (DMHCA) as high affinity ligands for RORγ. The oxysterols were unexpectedly able to reverse the antagonistic effect of a retinoid (e.g. ATRA) or rexinoid (e.g. LE540) at saturating concentration in a dose dependent fashion.

Furthermore, evidence was found for high affinity binding of $^3$H-labeled 25-hydroxycholesterol with an apparent displacement Ki of 20 - 2000 nM, depending on the assay conditions and the concentration of $^3$H-labeled 25-hydroxycholesterol to be displaced. In a specific radioactive displacement assay using $^3$H-25-hydroxycholesterol at saturating concentration (500nM), a dose-dependent competitive displacement by non-labelled 25-hydroxycholesterol, LE540, TTNPB or CH55 took place (see Fig. 1).

The identification of high affinity ligands for RORγ with agonistic and antagonistic properties is the basis to enable experts knowledgeable in the field to establish assays for the identification of novel agonistic and antagonistic RORγ ligands from libraries of small molecules. The identification of ligands which bind to and modulate the activity of RORγ1 and RORγ2 is the first mandatory step to develop new small molecule based medicines with a potential to be developed for the treatment of diseases which are directly or indirectly controlled by the activity of RORγ1 or RORγ2. Such diseases include but are not restricted to inflammatory diseases, rheumatoid arthritis, autoimmune diseases or diseases with an autoimmune componente such as Systemic Lupus Erythomatosis, Inflammatory Bowel Disease (Crohn's Disease), Ulcerative Colitis, Inflammatory Skin Diseases such as Atopic Eczema or Psoriasis, Multiple Sclerosis or similar diseases.

Thus, the present invention also relates to a method for identifying novel ligands of the RORgamma receptor.

Different assay systems can be used for the *de novo* identification of nuclear receptor ligands, in general. Such assay systems are either biochemical, cell-free assays that employ a purified receptor from a natural cell environment, or preferably, a purified recombinant version of the nuclear receptor under investigation, or they are cell-based assay systems. The nuclear receptor under investigation is usually employed either as a full-length receptor or as parts thereof that cover at least those amino acid residues that constitute the ligand-binding domain of the nuclear receptor. The ligand binding domain (LBD) is defined as that protein domain of a nuclear receptor that extends distal or C-terminally from the highly conserved Zinc-finger containing DNA binding domain and from the less conserved hinge region up to the C-terminus of the nuclear receptor.

For a biochemical assay the nuclear receptor or an LBD-containing part thereof is recombinantly expressed in one of the usual expression systems such as *E. coli*, yeast, baculovirus-induced insect cells or a mammalian cell culture system. The nuclear receptor expression construct can be either native, i.e. resembling entirely a naturally occurring amino acid sequence or, preferably, it can contain certain artificial amino acid stretches that resemble affinity tags for the ease of purification. Alternatively, the NR construct can be fused to another protein or protein domain that acts as an affinity or localization tag or as a folding and stabilization aid.

The recombinantly expressed NR protein is then purified using a standard method, which is available for those skilled in the art of protein expression and purification up to a degree, which allows characterization of its ligand binding activity. For the purpose of ligand screening, the recombinant NR protein can be used as such or it can be further labeled or decorated with labeling reagents. Unmodified proteins can be used in radioligand or fluorescent ligand displacement assays where a radiolabeled or fluorescently-tagged *bona fide* ligand is available as a reference ligand. For the use in FRET or Alphascreen® type assays, the recombinant NR protein must be decorated with further reagents such as an Europium-chelate fluorophore containing antibody directed against an affinity tag. Such decoration reagents emit a primary signal that can be further transferred, enhanced or complemented by a second reagent. In case of the FRET assay, the second reagent is a chromophore than can absorb light of the wavelength that is emitted by the primary reagent, which is attached to the NR protein. When a Eu-chelate is the primary fluorescent light source originating from the NR-decorated reagent, then i.e. Allophycocyanin (APC) can be the second fluorescent absorber. The Fluorescence Resonance Energy Transfer (FRET) effect only takes place when emitter and absorber come into close proximity.

Therefore, most non-radiolabeled biochemical nuclear receptor assays make use of the protein recruiting capabilities of nuclear receptors. Nuclear receptors, in general, tend to recruit certain adaptor proteins that tether them

to chromatin and transcriptional activity modifying protein complexes. In the absence of a ligand, most NRs recruit so-called corepressors that contain or further recruit Histone Deacetylase activity and hence keep the chromatin region around the NR response element transcriptionally silent. When an activating ligand, an agonist, binds to the nuclear receptor, the corepressors are replaced by co-activators, adaptor proteins that recruit other proteins with Histone-Acetylase activity. The resulting chromating opening can then result in increased transcriptional activity starting from these promoter regions.

Thus, the recruitment of co-activators is a critical step in the activation cascade initiated by an agonist ligand. Therefore biochemical assays system can detect ligand binding by detecting the recruitment of co-activators. This co-activator recruitment, in turn, can be detected if the co-activator is labeled by the secondary reagent required for this assay type. Nuclear Receptor ligands can be identified using a nuclear receptor-peptide interaction assay that utilizes time-resolved fluorescence resonance energy transfer (TR-FRET). This assay is based on the principle finding that ligands can induce conformational alterations upon binding within the ligand-binding domain (LBD) of nuclear receptors that alter interactions with coactivator or corepressor proteins which in turn mediate alterations in transcriptional activity. In TR-FRET, a fluorescent donor molecule transfers energy via dipole-dipole interaction to an, usually fluorescent, acceptor molecule. This technique is a standard spectroscopic technique for measuring distances and changes in distances in the 10-70 A range, which depends on the $R^{-6}$ distance between donor and acceptor molecule. Using europium cryptate in conjugation with the multichromophoric Allophycocyanine, interactions with a very large $R_0$ of 90 A can be achieved (Mathis et al., Clin. Chem. 1993, 39:1953-1959).

For the purpose of a nuclear receptor ligand sensitive FRET assay, one can attach the acceptor label to one of the known coactivators proteins, preferably to a peptide that is derived from a coactivator. Usually, a 20 to 30mer peptide that resembles one of the well-defined LXXLL-motifs that are responsible for the physical NR-cofactor interaction is sufficient. The fluorophore label can be attached to such a peptide by various means, including a biotinylation of the peptide and capture of the biotin by an e.g. streptavidin-APC complex.

In the case of RORgamma, a certain constitutive activity in such biochemical assay systems can be observed. This means just the NR together with cofactor peptides and all necessary reagents create a signal already. In the case of such constitutive active NRs it can be possible to further stimulate such signal by agonist compounds that further stabilize or enhance the active NR conformation. Compounds, however, that lead to a dose dependent signal decrease in the case of such constitutive active receptors are termed inversed agonists.

For the purpose of reducing the pro-inflammatory Th17 cell count in conjunction with amelioration of RORgamma mediated immunological diseases the identification of such inverse agonists is sought.

For cell-based nuclear receptor assays, mostly recombinant expressed versions of the nuclear receptor under investigation are used that are brought into the cell either by transient transfection or by transfection and subsequent selection into a stable nuclear receptor construct expressing cell line. Such cell lines that transiently or stably express a nuclear receptor or an LBD-containing part thereof can be used for ligand screening when they either harbor a plasmid where said nuclear receptor can initiate the transcription of a certain reporter gene under the control of a nuclear receptor constructs-specific DNA response element in the reporter promoter, or the nuclear receptor constructs which is transfected controls the transcription of a certain native target gene. Changes in endogenous or native target gene expression which are brought about by a certain ligand modulating the NR's transcriptional control over such target genes can be monitored with any target gene mRNA specific detection systems such as quantitative real time polymerase chain reaction (qRT-PCR) based methods like Taqman®, Light Cycler®, Sybr Green® incorporation or similar techniques. Examples for reporter genes whose expression levels can be directly monitored through monitoring the activity of the encoded reporter enzyme are Luciferases, Chloramphenicol-Acetyltransferases or similar well-defined reporter enzymes.

An agonist ligand in such a RORgamma cell-based reporter assay would stimulate the constitutive reporter signal further, an inverse agonist ligand would dose dependently reduce the reporter signal.

As described in more detail in the examples, a GST-RORgamma-LBD fusion protein is expressed and purified from *E. coli.* The TR-FRET assays were performed in a final volume of 25μl in individual wells of a 384 well plate using a Tris-based buffer system: 10 - 50mM Tris-HCl pH7.9; 50 - 100mM KCl, 1-10mM $MgCl_2$; 20 - 100ng/μl BSA), containing 20-60ng/well recombinantly expressed RORγ-LBD fused to GST, 200-600nM N-terminally biotinylated peptide (e.g. derived from SRC-1 or TIF-2), 50 - 500 ng/well Streptavidin-xlAPC conjugate (Prozyme) and 2-20ng/well Eu W1024 - antiGST (Perkin Elmer). The TR-FRET signal was detected using a Perkin Elmer VICTOR2V™ Multilabel Counter by detecting emitted light at 665 nm and 615 nm and the results plotted as the ratio of 665nm/615nm.

It is common understanding to those skilled in the art, that the RORgamma-LBD moiety in the GST fusion protein can be exchanged by RORgamma containing protein fragments which are smaller or larger (e.g. the fullength RORγ1 or RORγ2). Also the GST moiety can be exchanged by other affinity tags (e.g. His-tag, myc-tag, HA-tag) in combination with the respective Europium-labelled antibodies detecting the used affinity tags. In addition, the biotinylated peptide derived from the nuclear receptor interacting domains of coactivator proteins (e.g. SRC-1 and TIF-2) can be exchanged by larger fragments of said coactivators or even full-length coactivators which are recombinantly expressed in prokaryotic of eukaryotic systems and biotinylated *in vitro* or *in vivo.* The nuclear receptor-peptide interaction assay

described herein could also be performed using alternative detection methodologies such as Fluorescence Polarization (FP (WO 1999/027365) NUCLEAR HORMONE RECEPTOR DRUG SCREENS) and AlphaScreen using a Fusion Alpha Multilabel Reader (commercially available via PerkinElmer). For those skilled in the art, both fluorescently labelled peptides as well as a fluorescently labelled ligand could be used for detection of ligand interaction with RORgamma or ligand mediated interaction of a cofactor-derived peptide with RORgamma.

The following examples describe the invention in more detail. These examples, however, are not construed to limit the scope of the invention in any manner.

Examples

**Protein Expression and Purification**

For determination of a ligand-mediated cofactor peptide interaction to quantify ligand interaction with the nuclear receptor Retinoid Acid Receptor-related Orphan Receptor gamma (RORγ), the respective Ligand Binding Domain (LBD) of RORgamma was expressed in *E. coli* and purified as described below:

The human RORg ligand binding domain (LBD) was expressed in *E. coli* strain BL21 (DE3) as an N-terminal glutathione-S-transferase (GST) tagged fusion protein. The DNA encoding the RORg ligand-binding domain was cloned into vector pDEST15 (Invitrogen). The amino acid boundaries of the ligand-binding domain were amino acids 267-518 of Database entry NM_005060 (RefSeq). Expression in pDEST15 is controlled by an IPTG inducible T7 promoter and cloning and transformation of *E. coli* was done essentially according to standard protocols known to persons skilled in the art and supplied by Invitrogen.

Expression and purification of the RORγ-LBD: An overnight preculture of *E. coli* strain BL21 (DE3) (Invitrogen) transformed with pDEST15-huRORg-LBD was diluted 1:20 in LB-Ampicillin medium and grown at 30°C to an optical density of $OD_{600}$ = 0.6. Gene expression was then induced by addition of IPTG to an end concentration of 0.5 mM. Cells were incubated an additional 16h at 16°C, 180rpm. Cells were collected by centrifugation (7000xg, 10min, room temperature). Cells were re-suspended in 10ml lysis buffer (50mM Tris pH 7.5, 20mM NaCl, 5mM EDTA and 4mg/ml lysozyme) per gram wet pellet weight and left at room temperature for 30min. Subsequently, 1μl DNaseI solution (2mg/ml) per ml solution is added and $MgCl_2$ is added to 20mM final concentration and the resulting solution is incubated on ice for 15min. Cells were then subjected to sonication and cell debris removed via centrifugation (14000xg, 60min, 4°C). Per 1l of original cell culture 0.5ml prewashed Glutathione 4B sepharose slurry (Pharmacia) was added and the suspension kept slowly rotating for 1 h at 4°C. Glutathione 4B sepharose beads were pelleted by centrifugation (1000xg, 1 min, 4°C) and washed three times in wash buffer (25mM Tris, pH7.5, 50mM KCl, 4mM $MgCl_2$ and 1M NaCl). The pellet was re-suspended in 500μl elution buffer per gram wet pellet weight (elution buffer: 20mM Tris, pH7.5, 60mM KCl, 5mM $MgCl_2$ and 10mM glutathione added immediately prior to use as powder). The suspension was left rotating for 15min at 4°C, the beads pelleted and eluted again with 50mM glutathione in elution buffer. For subsequent TR FRET assays, glycerol was added to this protein solution to 10%(v/v).

For the radioligand displacement assay (Example 3), the eluate was dialysed overnight in 20mM Tris-HCl buffer (pH 7.5) containing 60mM KCl, 5mM $MaCl_2$ and used directly in the assay.

Determination of the interaction of a ligand with the human RORgamma ligand-binding domain was done by using a Ligand Sensing Assay based on Time-resolved Fluorescence Energy Transfer (TR-FRET).

**TR-FRET Activity Assay**

This method measures the ability of putative ligands to modulate the interaction between the purified bacterial expressed RORg ligand binding domain (LBD) and synthetic N-terminally biotinylated peptides which are derived from nuclear receptor coactivator proteins such as but not limited to SRC1 (NcoA1), SRC2 (NcoA2,TIF2), SRC3 (NcoA3), PGC1α, PGC1β, CBP, GRIP1, TRAP220, RIP140. The peptides used for Example 1 and Example 2 are listed in Table 1 below:

**Table 1**

| Peptide Name (aa range) | DB entry Protein | DB entry DNA | Sequence |
|---|---|---|---|
| **SRC1(676-700)** | NP_003734 | NM_003743 | NH2-CPSSHSSLTERHKILHRLLQEGSPS-COOH |
| **TIF2(628-658)** | NP_006531 | NM_006540 | NH2-GQSRLHDSKGQTKLLQLLTTKSDQ-COOH |

The ligand-binding domain (LBD) of RORγ was expressed as fusion protein with GST in BL-21 (BL3) cells using the vector pDEST15. Cells were lysed by lysozyme-treatment and sonication, and the fusion proteins purified over glutathione sepharose (Pharmacia) according to the manufacturers instructions. For screening of compounds for their influence on the RORγ-peptide interaction, the LANCE technology (Perkin Elmer) was applied. This method relies on the binding dependent energy transfer from a donor to an acceptor fluorophor attached to the binding partner of interest. For ease of handling and reduction of background from compound fluorescence LANCE technology makes use of generic fluorophore labels and time resolved detection assays were done in a final volume of 25μl in a 384 well plate, in a Tris-based buffer (20mM Tris-HCl pH7.9; 60mM KCl, 5mM $MgCl_2$; 35ng/μl BSA), containing 20-60ng/well recombinantly expressed RORγ-LBD fused to GST, 200-600nM N-terminally biotinylated peptide, 200 ng/well Streptavidin-xlAPC conjugate (Prozyme) and 6-10ng/well Eu W1024-antiGST (Perkin Elmer). DMSO content of the samples was kept at 1%. After generation of the assay mix the potentially RORγ modulating ligands were diluted. After his step, the assay was equilibrated for one hour in the dark at room temperature in FIA-plates black 384 well (Greiner). The LANCE signal was detected by a Perkin Elmer VICTOR2V™ Multilabel Counter. The results were visualized by plotting the ratio between the emitted light at 665 nm and 615 nm. A basal level of RORγ-peptide formation is observed in the absence of added ligand. Ligands that promote the complex formation induce a concentration-dependent increase in time-resolved fluorescent signal. Compounds which bind equally well to both monomeric RORγ and to the RORγ-peptide complex would be expected to give no change in signal, whereas ligands, which bind preferentially to the monomeric receptor would be expected to induce a concentration-dependent decrease in the observed signal.

Example 1:

To assess the agonistic and antagonistic potential of the compounds, $EC_{50}$ or $IC_{50}$-values were determined using a Ligand Sensing Assay based on Time-resolved Fluorescence Energy Transfer (TR-FRET) as described above. The normalised TR FRET assay values, using the following equation: 1000 * 655nm measurement value / 615nm measurement value, were transferred to the program GraphPad Prism to generate graphs and dose response curves using the following equation:

Equation: Sigmoidal dose-response (variable slope)

Y=Bottom + (Top-Bottom)/(1+10^((LogEC50-X)*HillSlope))

X is the logarithm of the concentration. Y is the response.
Y starts at Bottom and goes to Top with a sigmoidal shape.

This is identical to the "four parameter logistic equation". The $EC_{50}$ or IC50 values are calculated using this equation.

Examples for selected compounds are listed in Table 2 below:

**Table 2:**

| Compound | | Peptide | $IC_{50}$ [nM] |
|---|---|---|---|
| LE540 | antagonistic | SRC1(676-700) | 3600 |
| LE540 | antagonistic | TIF2(628-658) | 4900 |
| LE135 | antagonistic | SRC1 (676-700) | 19300 |
| LE135 | antagonistic | TIF2(628-658) | 13200 |
| Am580 | antagonistic | SRC1(676-700) | 23400 |
| Am580 | antagonistic | TIF2(628-658) | 19800 |
| Ch55 | antagonistic | SRC1(676-700) | 12200 |
| Ch55 | antagonistic | TIF2(628-658) | 11300 |
| TTNPB | antagonistic | SRC1(676-700) | 8600 |
| TTNPB | antagonistic | TIF2(628-658) | 8200 |
| 9cis RA | antagonistic | SRC1(676-700) | 14000 |

(continued)

| Compound | | Peptide | $IC_{50}$ [nM] |
|---|---|---|---|
| 9cis RA | antagonistic | TIF2(628-658) | 15900 |
| ATRA | antagonistic | SRC1(676-700) | 18300 |
| ATRA | antagonistic | TIF2(628-658) | 14200 |
| | | | |
| **Compound** | | **Peptide** | **$EC_{50}$ [nM]** |
| 22R-Hydroxycholesterol | agonistic | TIF2(628-658) | 16.5 |
| 25-Hydroxycholesterol | agonistic | TIF2(628-658) | 18.6 |
| (25R)-26-Hydroxycholesterol | agonistic | TIF2(628-658) | 14.1 |
| Cholenic Acid Methyl Ester | agonistic | TIF2(628-658) | 27.5 |
| DMHCA | agonistic | TIF2(628-658) | 10.7 |

All compounds listed above with retinoid structures (LE540, LE135, Am580, Ch55, TTNPB, 9cisRA and ATRA) do reduce the signal in the TR-FRET assay in a dose dependent fashion with IC50 values ranging from 3600 nM for LE540 to 23400 nM for Am580 using SRC1 as interacting peptide.

In contrast, the oxysterol compounds 22R-hydroxycholesterol, 25-hydroxycholesterol, (25R)-26-hydroxycholesterol, cholenic acid methyl ester and DMHCA do act agonistic in a highly potent fashion with EC50 values ranging from 10.7 nM for DMHCA to 27.7 nM for cholenic acid methyl ester with TIF2 as interacting peptide.

Example 2:

In a variation of the assay described above, an antagonistic compound was added in a saturating concentration (4μM) to the assay mix. Then the agonistic compounds were diluted and the assay was equilibrated for one hour in the dark. Titration of agonists on top of saturating antagonist concentration still produced dose response curves for the agonists, but with higher apparent $EC_{50}$-values for the agonists compared to the data obtained in absence of antagonist (see Table 2). This shows that the antagonist can be replaced by the agonists. The apparent $EC_{50}$-values were as listed below in Table 3:

**Table 3**

| Compound (assay includes 4μM LE540) | | Peptide | $EC_{50}$ [nM] |
|---|---|---|---|
| 22R-Hydroxycholesterol | agonistic | TIF2(628-658) | 154 |
| 25-Hydroxycholesterol | agonistic | TIF2(628-658) | 363 |
| (25R)-26-Hydroxychol esterol | agonistic | TIF2(628-658) | 146 |
| Cholenic Acid Methyl Ester | agonistic | TIF2(628-658) | 311 |
| DMHCA | agonistic | TIF2(628-658) | 85 |

In this variation of the TR-FRET assay, the oxysterol-type compounds do displace an antagonistic compound at saturating concentration (4μM) in a dose dependent way with calculated EC50 values ranging from 85 nM for DMHCA and 363 nM for 25-Hydroxycholesterol using TIF2 as interacting peptide. The results demonstrate the unexpected finding that these oxysterol compounds act as highly potent agonistic RORγ ligands.

Example 3:

**Radioligand Displacement Assay**

This method measures the ability of putative ligands to displace a radioactively labeled compound that is bound to the RORgamma ligand-binding domain.

The ligand-binding domain (LBD) of RORγ was expressed as fusion protein with GST in BL-21 cells using the

vector pDEST15. Cells were lysed by lysozyme-treatment and sonication, and the fusion proteins purified over glutathione sepharose (Pharmacia) according to the manufacturers instructions. For screening of compounds for their ability to bind to RORγ, commercially available 25-[26,27-$^3$H]-hydroxycholesterol (Perkin Elmer, NET674250UC), was bound to the protein and displacement by nonradioactive ligands was observed. The assay was done in a final volume of 100μl in a 96-well glutathione and scintillant coated microplate (Perkin Elmer, SMP109001 PK). 50-200ng/well recombinantly expressed RORγ-LBD fused to GST was incubated with 100nM 25-[26,27-$^3$H]-hydroxycholesterol and 400nM 25-hydroxycholesterol in a Tris-based buffer (20mM Tris-HCl pH7.9; 60mM KCl, 5mM $MgCl_2$; 45ng/μl BSA). Compounds to be tested were titrated and added to the protein-radioligand mix. DMSO content of the samples was kept at 1 %. After addition of the compounds, the assay was equilibrated for 30min at room temperature. After this incubation the assay plate wells were washed twice with Tris buffer (20mM Tris-HCl pH7.5; 60mM KCl, 5mM $MgCl_2$) and subsequently measured for 500sec per well in a LUMIstar OPTIMA (BMG).

The results of the assay are shown in Figure 1. The retinoid like structures, LE540, TTNBP and Ch55 but also the oxysterol 25-hydroxycholesterol are, in a dose dependent fashion, able to displace 25-[26,27-$^3$H]-hydroxycholesterol, which was prebound to RORgamma ligand binding domain. The apparent potency and efficacy of displacement are highest with LE540 and 25-hydroxycholesterol. TTNBP is less potent but seems rather efficient while Ch55 shows the lowest potency and efficacy in this displacement assay allowing to distinguish among RORgamma interacting ligands.

**Claims**

1. A RORgamma modulator for use in the treatment or prophylaxis of a disease or disorder associated with the inhibition or activation of a RORgamma receptor.

2. The RORgamma modulator according to claim 1, wherein the RORgamma receptor activity is reduced.

3. The RORgamma modulator according to claim 2, wherein the disease or disorder is selected from the group consisting of autoimmune diseases, inflammatory skin diseases and multiple sclerosis.

4. The RORgamma modulator according to claim 3, wherein the autoimmune diseases are selected from Rheumatoid Arthritis, Systemic Lupus Erythomatosis, Inflammatory Bowel Disease (Crohn's Disease) and Ulcerative Colitis.

5. The RORgamma modulator according to claim 3, wherein the inflammatory skin diseases are selected from Atopic Eczema and Psoriasis.

6. The RORgamma modulator according to any of claims 1 to 5, which comprises a compound of formula (I)

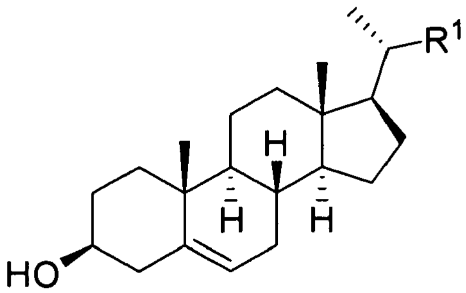

(I)

or a solvate or a pharmaceutically acceptable salt thereof, wherein

$R^1$ is

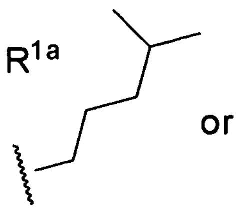

or

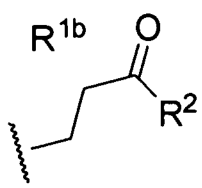

or

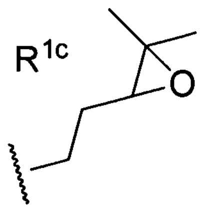

wherein each $R^{1a}$, $R^{1b}$ or $R^{1c}$ is optionally substituted with one or two hydroxy groups,
$R^2$ is $OR^3$ or $NR^3R^4$, and
$R^3$ and $R^4$ are independently from each other selected from hydrogen or $C_1$-$C_3$ alkyl.

7. The RORgamma modulator according to any of claims 1 to 5, which comprises a compound of formula (II)

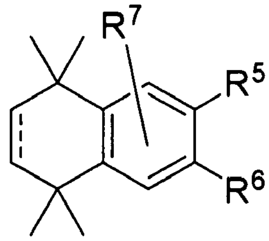

(II)

or a solvate or a pharmaceutically acceptable salt thereof
wherein

$R^5$ is $CONHR^8$, $NHCOR^8$, $C(O)R^8$, $CH=CHR^8$, $C(CH_3)=CHR^8$, $C\equiv CR^8$, $CH(OH)CH=CHR^8$, $C(O)CH=CHR^8$, 5 to 6-membered heterocyclyl-$R^8$,
$R^6$ is hydrogen,
$R^5$ and $R^6$ may also together form

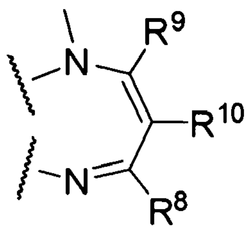

$R^7$ is hydrogen, fluorine, chlorine or hydroxy,
$R^8$ is 4-yl-benzoic acid or 6-yl-2-naphthoic acid,
$R^9$ and $R^{10}$ are hydrogen or $R^9$ and $R^{10}$ form together with the bond to which they attach a fused 5 to10-membered heteroaromatic or aromatic monocyclic or bicyclic ring.

**8.** Use of a RORgamma modulator for the preparation of a medicament for treating or preventing a disease or disorder associated with the inhibition or activation of a RORgamma receptor.

**9.** The compounds (Z)-4-(10,10,13,13,15-pentamethyl-11,12,13,15-tetrahydro-10H-dinaphtho[2,3-b:1',2'-e][1,4]diazepin-7-yl)benzoic acid (LE540), (Z)-4-(5,7,7,10,10-pentamethyl-7,8,9,10-tetrahydro-5H-benzo[e]naphtho[2,3-b][1,4]diazepin-13-yl)benzoic acid (LE135), (E)-4-(2-(5,5,8,8-tetramethyl-5,6,7,8-tetrahydronaphthalen-2-yl)prop-1-enyl)benzoic acid (TTNBP), and (E)-4-(3-(3,5-di-tert-butylphenyl)-3-oxoprop-1-enyl)benzoic acid (Ch55) as a RORgamma modulators.

**10.** 22-R-Hydroxycholesterol, 25-Hydroxycholesterol, (25R)-26-Hydroxycholesterol, 5-cholenic acid-methyl-ester-3β-hydroxycholenamide (CHAME) and N,N-dimethyl-3-hydroxycholenamide (DMHCA) as RORgamma modulators.

**11.** A method for identifying a modulator of RORgamma activity in a biochemical cell-free *in vitro* assay system comprising the steps of

(a) administrating to such assay system an effective amount of a RORgamma modulator according to any of the claims 6 to 7 or 9 to 10, sufficient to induce or reduce the readout of RORgamma activity in said biochemical assay system and
(b) comparing the measured activity with the activity of a reference RORgamma modulator.

**12.** The method of claim 12, wherein the biochemical assay system is selected from a homogenous time-resolved fluorescence resonance energy transfer (HTR-FRET) assay, a radioligand binding and a displacement assay in conjunction with a recombinantly expressed RORgamma protein of the sequence SEQ ID NO 1, and a coactivator

peptide with a sequence of SEQ ID NO 2 or 3.

**13.** The method of any of claims 11 to 13, wherein the reference RORgamma modulator is one or more of the compounds claimed in claims 6 to 7 or 9 to 10 and is used as a control to monitor the activity of newly to be identified modulators.

# Figure 1

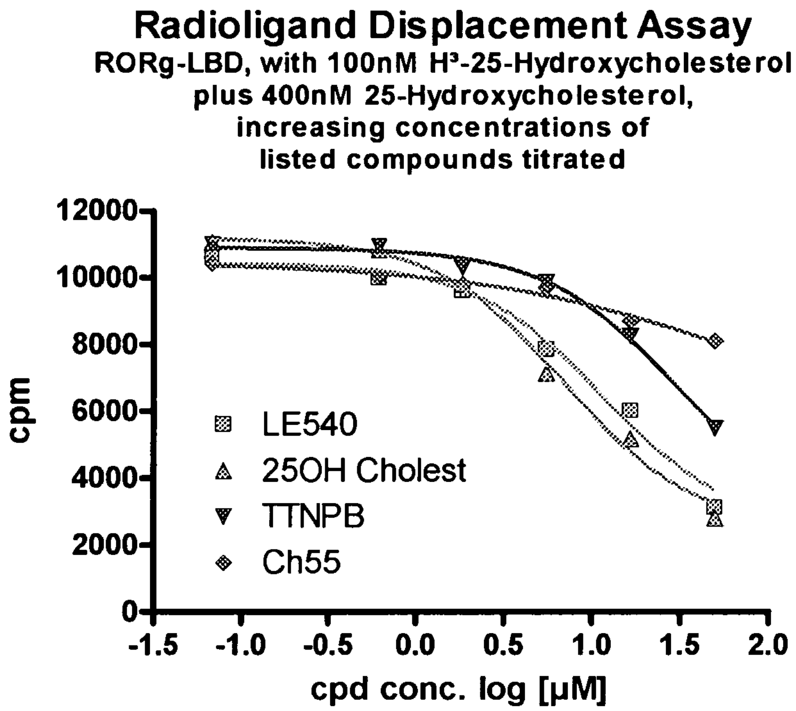
Radioligand Displacement Assay
RORg-LBD, with 100nM H³-25-Hydroxycholesterol
plus 400nM 25-Hydroxycholesterol,
increasing concentrations of
listed compounds titrated
12000
10000
8000
6000
4000
2000
0
cpm
LE540
25OH Cholest
TTNPB
Ch55
-1.5
-1.0
-0.5
0.0
0.5
1.0
1.5
2.0
cpd conc. log [μM]

# Figure 2

| | |
|---|---|
| LE540 | 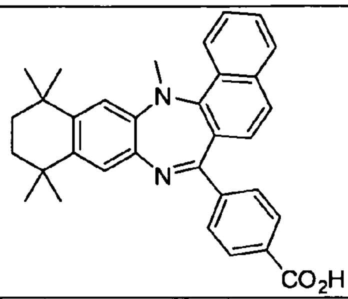 |
| LE135 | 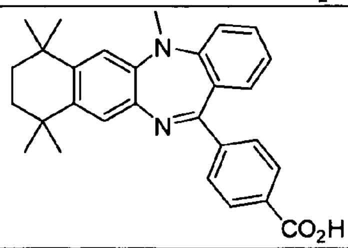 |
| AM580 | 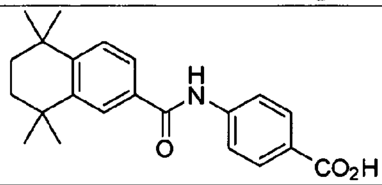 |
| CH55 | 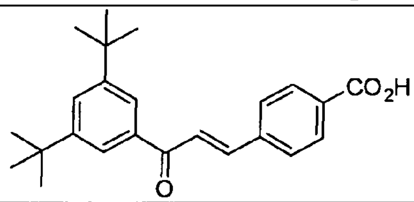 |
| TTNPB | 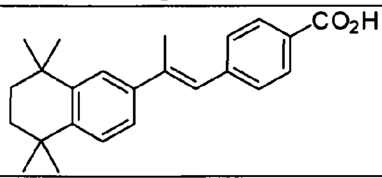 |
| 9cisRA | 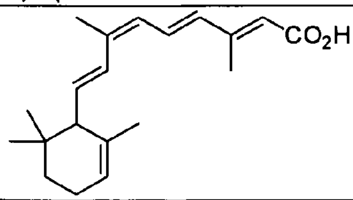 |
| ATRA | 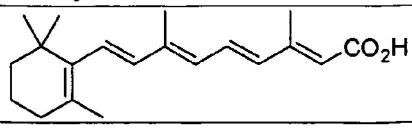 |

| | |
|---|---|
| (22R)-Hydroxycholesterol<br>CAS 17954-98-2<br><br>Synonyms:<br>(22R)-22-Hydroxycholesterol<br>(22R)-Cholest-5-ene-3β,22-diol<br>22α-Hydroxycholesterol<br>Narthesterol | 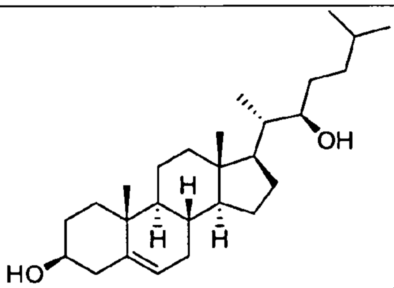 |
| 25-Hydroxycholesterol<br>CAS 2140-46-7<br><br>Synonyms:<br>Cholest-5-ene-3.beta.,25-diol (8CI);<br>5-Cholesten-3.beta.,25-diol | 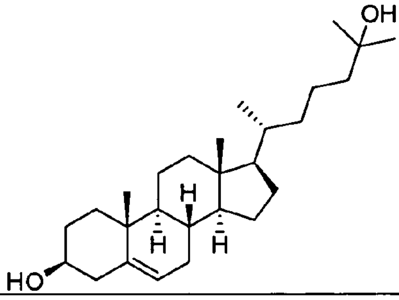 |
| (25R)-26-Hydroxycholesterol<br>CAS 20380-11-4<br><br>Synonyms:<br>Cholest-5-ene-3β,26-diol, (25R)- (8CI)<br>(25R)-Cholest-5-ene-3β,26-diol | 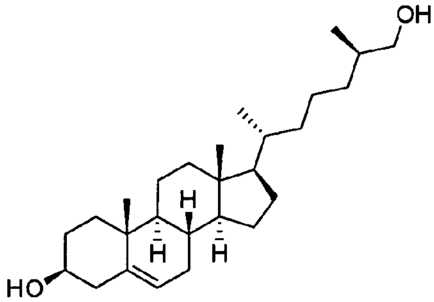 |
| 3β-hydroxy-5-cholenic acid methyl ester | 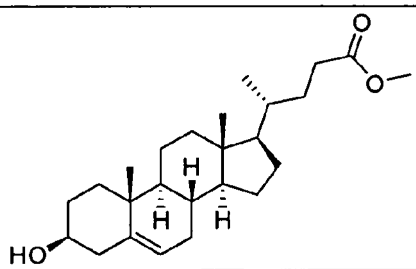 |
| DMHCA<br>CAS 79066-03-8 | 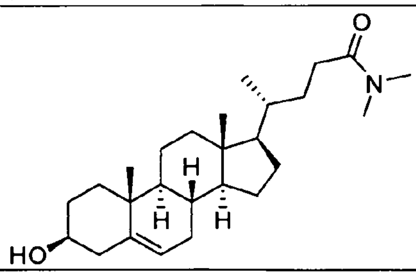 |

Europäisches Patentamt
European Patent Office
Office européen des brevets

# EUROPEAN SEARCH REPORT

Application Number
EP 08 01 8795

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/115469 A (UNIV CALIFORNIA [US]; PARHAMI FARHAD [US]) 25 September 2008 (2008-09-25) * page 3, paragraph 4 * * claim 7 * * page 14, paragraph 1 * ----- | 1-4,6,8, 10 | INV. A61K31/575 A61P17/06 A61P37/06 A61P1/00 A61P17/00 A61P29/00 G01N33/50 |
| X | HELIOVAARA M ET AL: "Serum cholesterol and risk of rheumatoid arthritis in a cohort of 52800 men and women" BRITISH JOURNAL OF RHEUMATOLOGY, BAILLIERE TINDALL, LONDON, GB, vol. 35, no. 3, 1 January 1996 (1996-01-01), pages 255-257, XP008101938 ISSN: 0263-7103 * abstract * ----- | 1-4,6,8, 10 | |
| | | | TECHNICAL FIELDS SEARCHED (IPC) |
| | | | A61K |

~~The present search report has been drawn up for all claims~~

| Place of search | Date of completion of the search | Examiner |
|---|---|---|
| The Hague | 18 February 2009 | Strack, Eberhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

6
EPO FORM 1503 03.82 (P04C01)

Europäisches Patentamt
European Patent Office
Office européen des brevets

Application Number

EP 08 01 8795

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

see annex

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION
## SHEET B

Application Number
EP 08 01 8795

The Search Division considers that the present European patent application does not comply with the requirements of unity of invention and relates to several inventions or groups of inventions, namely:

1. claims: 1-4,6,8,10 (partially)

   RORgamma modulator which is a compound of Formula I/R1a (in particular 22R- or 25-hydroxycholesterol) for use in the treatment or prophylaxis of rheumatoid arthritis

   ---

2. claims: 1-4,6,8,10 (partially)

   RORgamma modulator which is a compound of Formula I/R1b for use in the treatment or prophylaxis of rheumatoid arthritis

   ---

3. claims: 1-4,6,8 (partially)

   RORgamma modulator which is a compound of Formula I/R1c for use in the treatment or prophylaxis of rheumatoid arthritis

   ---

4. claims: 1-4,7-9 (partially)

   RORgamma modulator which is a compound of Formula II for use in the treatment or prophylaxis of rheumatoid arthritis

   ---

5. claims: 1-4,8,9 (partially)

   RORgamma modulator which is CH55 for use in the treatment or prophylaxis of rheumatoid arthritis

   ---

6. claims: 1-4,6-9 (partially)

   RORgamma modulator for use in the treatment or prophylaxis of systemic lupus e.

   ---

7. claims: 1-4,6-9 (partially)

   RORgamma modulator for use in the treatment or prophylaxis of Morbus Crohn

   ---

8. claims: 1-4,6-9 (partially)

   RORgamma modulator for use in the treatment or prophylaxis of colitis ulcerosa

   ---

9. claims: 1-3,6-9 (partially); 5 (completely)

Europäisches Patentamt
European Patent Office
Office européen des brevets

**LACK OF UNITY OF INVENTION SHEET B**

Application Number

EP 08 01 8795

The Search Division considers that the present European patent application does not comply with the requirements of unity of invention and relates to several inventions or groups of inventions, namely:

RORgamma modulator for use in the treatment or prophylaxis of inflammatory skin diseases

---

10\. claims: 1-3,6-9 (partially)

RORgamma modulator for use in the treatment or prophylaxis of multiple sclerosis

---

11\. claims: 11-13 (completely)

Method according to claims 11-13

---

## ANNEX TO THE EUROPEAN SEARCH REPORT ON EUROPEAN PATENT APPLICATION NO. EP 08 01 8795

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2009

| Patent document cited in search report | Publication date | Patent family member(s) | Publication date |
|---|---|---|---|
| WO 2008115469 A | 25-09-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200024757 A
- WO 1999027365 A


### Non-patent literature cited in the description


- **Becker-Andree.** *Biochem. Biophys. Res. Commun.,* 1993, vol. 194, 1371-1379
- **Andre et al.** *Gene,* 1998, vol. 516, 277-283
- **He et al.** *Immunity,* 1998, vol. 9, 797-806
- **Mangelsdorf et al.** *Cell,* 1995, vol. 83, 835-839
- **McKenna et al.** *Endocrine Rev.,* 1999, vol. 20, 321-344
- **Vanacker et al.** *Mol. Endocrinol.,* 1999, vol. 13, 864-773
- **Willy et al.** *PNAS,* 2004, vol. 101, 8912-8917
- **Andre et al.** *Gene,* 1998, vol. 216, 277-283
- **Villey et al.** *Eur. J. Immunol.,* 1999, vol. 29, 4072-7080
- **Eberl et al.** *Science,* 2004, vol. 305, 248-251
- **Evans et al.** *Science,* 1988, vol. 240, 889-895
- **Giguere et al.** *Genomics,* 1995, vol. 28, 596-598
- **Vanacker et al.** *Mol. Endocrinol.,* 1999, vol. 13, 764-773
- **Wilson et al.** *Mol. Cell Biol.,* 1993, vol. 13, 5794-5708
- **Forman et al.** Cross-talk among ROR alpha 1 and the Rev-erb family of orphan nuclear receptors. *Mol. Endocrinol.,* 1994, vol. 8, 1253-1261
- **Akashi ; Takumi.** *Nat. Struct. Mol. Biol.,* 2005, vol. 12, 441-448
- **Hamilton et al.** *Nature,* 1996, vol. 379, 736-739
- **Lau et al.** *J. Biol. Chem.,* 2008, vol. 283, 18411-18421
- **Andre et al.** *EMBO. J.,* 1998, vol. 17, 3867-3877
- **Eberl ; Littmann.** *Immunol. Rev.,* 2003, vol. 195, 81-90
- **Sun et al.** *Science,* 2000, vol. 288, 2369-2373
- **He et al.** *J. Immunol.,* 2000, vol. 164, 5668-5674
- **Tilley et al.** *J. Immunol.,* 2007, vol. 178, 3208-3218
- **Tesmer et al.** *Immunol. Rev.,* 2008, vol. 223, 87-113
- **Yang et al.** *Immunity,* 2008, vol. 28, 29-39
- **Gu et al.** *Eur. J. Immunol.,* 2008, vol. 38, 1807-1813
- **Kang et al.** *Physiol. Genomics,* 2007, vol. 31, 281-294
- **Wada et al.** *Exp. Biol. Medicine,* 2008
- **Kallen et al.** *Structure,* 2002, vol. 10, 1697-1707
- **Missbach et al.** *J. Biol. Chem.,* 1998, vol. 271, 13515-13522
- **Wiesenberg et al.** *Nucleic Acid Res.,* 1995, vol. 23, 327-333
- **Stehlin-Gaon et al.** *Nat. Struct. Biol.,* 2003, vol. 10, 820-825
- **Medvedev et al.** *Gene,* 1996, vol. 181, 199-206
- **Tesmer.** *Immunol. Rev.,* 2008, vol. 223, 97-113
- **Mathis et al.** *Clin. Chem.,* 1993, vol. 39, 1953-1959

SEQUENCE LISTING

<110> Phenex Pharmaceuticals AG

<120> Methods of identification of novel ligands for modulation of orphan nuclear receptor RAR-related orphan receptor-gamma (NR1F3) activity

<130> EP60913KG030pau

<140> not yet assigned
<141> 2008-10-28

<160> 3

<170> PatentIn version 3.3

<210> 1
<211> 260
<212> PRT
<213> Homo sapiens

<220>
<223> RORgamma protein sequence used in FRET and M1H assay types representing aa259-518 of RORgamma (NP_005051.2)

<400> 1

Thr Pro Glu Ala Pro Tyr Ala Ser Leu Thr Glu Ile Glu His Leu Val
1 5 10 15

Gln Ser Val Cys Lys Ser Tyr Arg Glu Thr Cys Gln Leu Arg Leu Glu
20 25 30

Asp Leu Leu Arg Gln Arg Ser Asn Ile Phe Ser Arg Glu Glu Val Thr
35 40 45

Gly Tyr Gln Arg Lys Ser Met Trp Glu Met Trp Glu Arg Cys Ala His
50 55 60

His Leu Thr Glu Ala Ile Gln Tyr Val Val Glu Phe Ala Lys Arg Leu
65 70 75 80

Ser Gly Phe Met Glu Leu Cys Gln Asn Asp Gln Ile Val Leu Leu Lys
85 90 95

Ala Gly Ala Met Glu Val Val Leu Val Arg Met Cys Arg Ala Tyr Asn
100 105 110

Ala Asp Asn Arg Thr Val Phe Phe Glu Gly Lys Tyr Gly Gly Met Glu
115 120 125

Leu Phe Arg Ala Leu Gly Cys Ser Glu Leu Ile Ser Ser Ile Phe Asp
130 135 140

Phe Ser His Ser Leu Ser Ala Leu His Phe Ser Glu Asp Glu Ile Ala
145 150 155 160

Leu Tyr Thr Ala Leu Val Leu Ile Asn Ala His Arg Pro Gly Leu Gln

165 170 175

Glu Lys Arg Lys Val Glu Gln Leu Gln Tyr Asn Leu Glu Leu Ala Phe
180 185 190

His His His Leu Cys Lys Thr His Arg Gln Ser Ile Leu Ala Lys Leu
195 200 205

Pro Pro Lys Gly Lys Leu Arg Ser Leu Cys Ser Gln His Val Glu Arg
210 215 220

Leu Gln Ile Phe Gln His Leu His Pro Ile Val Val Gln Ala Ala Phe
225 230 235 240

Pro Pro Leu Tyr Lys Glu Leu Phe Ser Thr Glu Thr Glu Ser Pro Val
245 250 255

Gly Leu Ser Lys
260

<210> 2
<211> 25
<212> PRT
<213> Artificial

<220>
<223> SRC1(676-700) peptide

<400> 2

Cys Pro Ser Ser His Ser Ser Leu Thr Glu Arg His Lys Ile Leu His
1 5 10 15

Arg Leu Leu Gln Glu Gly Ser Pro Ser
20 25

<210> 3
<211> 24
<212> PRT
<213> Artificial

<220>
<223> TIF2(628-658) peptide

<400> 3

Gly Gln Ser Arg Leu His Asp Ser Lys Gly Gln Thr Lys Leu Leu Gln
1 5 10 15

Leu Leu Thr Thr Lys Ser Asp Gln
20